# EUROPEAN PATENT APPLICATION

(11) **EP 4 062 948 A1**
(43) Date of publication of application: **28.09.2022**
(21) Application number: 21164144.4
(22) Date of filing: 23.03.2021
(51) Int. Cl.: A61L 9/20, A62B 7/10

(54) **PERSONAL APPARATUS FOR AIR DISINFECTION BY UV**

(71) Applicant: Respiray OÜ, 13522 Tallinn (EE)
(72) Inventor: Frorip, Aleksandr, 61508 Elva (EE); Slivinskiy, Evgeny, 00940 Helsinki (FI); Taveter, Aare, 11620 Tallinn (EE); Rennel, Annamaria, 42206 Oonurme (EE)
(74) Representative: Werhahn, Jasper Carl

(57) **Abstract**

The invention relates to a wearable apparatus for breathing air disinfection, the apparatus comprising:
- a wearable main body forming at least one ambient air duct which has an inlet end and an outlet end, which is configured to be worn by a human user on her/his shoulder or neck, and, when in use, the outlet of the air duct is below the chin of the user, wherein the air duct is configured such that it guides the air flow generated by the at least one fan at least to mouth and nose of the user,
- at least one UV-C radiation emitter, arranged within the air duct or at an end thereof such that it emits UV-C radiation into the air duct,
- at least one fan which is arranged within the air duct or at one end thereof, which generates an airflow from the inlet end to the outlet end,
- a power supply unit for supplying electrical power to the at least one UV-C emitter and at least one fan, arranged within or on the main body, and
a control unit for controlling the operation of the at least one UV-C emitter and at least one fan, arranged within or on the main body.

## Description

The invention relates to a wearable apparatus for breathing air disinfection.

Most of current protection devices for protecting humans against airborne infections have the shape of masks covering the lower part of a user's face and are based on the idea of preventing pathogens from being inhaled with dust particles or minor water droplets by means of mechanical filters.

Such filters can become dusty, damp, and therefore obstruct the passage of air through the filter, resulting in the unintentional inhalation of leakage air from around the filter. This allows pathogens to reach the mouth and nose of the user, notwithstanding that he wears such mask. It is a further important drawback of such masks, that wearing them is inconvenient for the user and, in particular for users which wear glasses or which have to work hard or are dealt with intense sports activities.

Regarding the physical principle of achieving a disinfection of the breathing air, besides mechanical filters recently devices have become known and came into commercial use, which are based on the idea that short wavelength (UV-C) ultraviolet radiation is very effective in destroying pathogens, in particular viruses. At the same time, such UV treatment does not destroy antigens of pathogens, that can cause an immune response in the body of a human.

Such device, which can be worn by a user for example within a face mask, has been disclosed in DE 20 2020 101 798 U1.

WO 2008/120 005 A1 discloses a device which protects the user from infection and which may simultaneously immunize the user against future infection and which also comprises a face mask. The device comprises a disinfection chamber, in which an incoming fluid (air) is disinfected and then discharged from the chamber e.g. in the face mask.

Besides other physical principles, UV irradiation is mentioned as a method to obtain the disinfection and/or sterilization of the fluid within the chamber. More specifically, the usage of UV-C radiation is specified in that document, and a variety of lamps for generating such radiation is mentioned. The document also discloses that the device comprising the disinfection chamber is portable and can be attached to a user via a belt or strap or can be worn in her/his hand.

In WO 2016/086 273 A1 a personal entertainment respiratory apparatus is disclosed, which provides air to a user to provide an immersive entertainment experience. The device can be configured to direct a flow of air within an ambient breathing proximity of the user. The device can comprise a controller and sensory particle dispenser and it can, besides other configurations, comprise a scarf, a gooseneck, a thin tube worn on the chest of a user etc., each configuration provided with a plurality of nozzles to direct the air to the mouth and nose of the user, preferably as an air curtain. In all those configurations, the major components of the apparatus are arranged in a device housing which is separate from that item through which the air is discharged to the user and connected thereto by means of a flexible tube.

The device of that document can contain a cleaning and filtering component for filtering particles from the air prior to providing the flow of air to the user. It is also mentioned that the filter can be capable of removing bacteria and viruses from the ambient air.

It is an object of the invention, to provide an apparatus for providing disinfected breathing air to a human user, which apparatus can easily and conveniently be worn by the user, has a simple and inexpensive construction and has a high efficiency in protecting the user from inhaling pathogens.

This and other objects are solved by an apparatus according to claim 1. Advantageous embodiments of the invention are subject of the dependent claims.

It is an idea of the invention, to present an apparatus which has all components integrated in a single device body (main body), without requiring plural parts and respective connecting elements. It is a further idea of the invention *not* to provide a mask-type device - with the well-known, above-mentioned drawbacks - but to propose an apparatus which discharges disinfected ambient air actively, i.e. not just by the respiration of the user, to the user's mouth and nose without covering them. In an embodiment of the invention, this is achieved by protecting the mouth and nose from un-disinfected air by generating an air curtain flowing along the user's face.

For providing the active discharge of disinfected air to the user's face, the apparatus comprises means for generating an airflow, typically configured as at least one fan or blower, respectively. In inexpensive configurations, the apparatus comprises a single fan arranged within a single air duct or at the inlet end thereof. However, configurations of the apparatus are possible - and can be preferred for special applications - which have more than one air duct within the device housing, and more than one fan or blower.

For disinfecting or sterilizing the ambient air, at least one UV-C emitter, in particular at least one UV-C emitting LED, is arranged within the air duct such that it emits UV-C radiation into the air duct. Typically, in low-cost embodiments of the apparatus a single UV-C LED will be arranged within a single air duct. However, for special applications and/or in high-end implementations, within a single air duct plural UV-C LEDs can be arranged, to irradiate the airflow within the air duct over a longer flow path and/or with higher intensity, than can be reached with a single LED. Besides this, configurations of the apparatus are possible, which have plural air ducts, each of which is provided with a single UV-C LED.

The apparatus comprises an integrated power supply unit for the UV-C emitter and the fan/s or blower/s. Preferably, the power supply unit comprises a rechargeable battery and an integrated charging socket, for charging the battery over a charging cable, in particular of the USB type.

Furthermore, the apparatus comprises an integrated control unit for controlling the operation thereof. This control unit can be configured as simple actuator means for manually controlling the operation of the apparatus, e.g. an on/off switch, and a simple indicator for informing the user of the operation status of the apparatus and the charge status of the battery. In more sophisticated configurations, however, the control unit can comprise plural actuator means and/or more complex control means and more complex display means, to select between several possible operation modes of the apparatus, in particular of the UV-C emitter/s and the fan/s.

In further embodiments, the apparatus comprises at least one of the following sensors: UV-C light sensor, airflow sensor, humidity sensor, power supply voltage sensor, and temperature sensor, and the control unit is adapted to receive and process signals from the at least one sensor, to control the operation of the apparatus, in particular of the at least one UV-C emitter and the at least one fan.

In a preferred embodiment of the invention, the main body is configured, when in use, to extend from the chin to the chest and/or at least one shoulder or the back of the user, and the inlet opening is located close to the chest or shoulder or back of the user. Preferably, the main body is configured to rest, in an upright position of the user, on her/his shoulder or neck. In a further embodiment, the main body comprises fixation means, such as belts or straps, for fixing it to the shoulder or neck of the user even in a non-upright position.

In a further preferred embodiment, at least that portion of the main body which is close to the chin of the user has a curved lateral cross-sectional shape to adapt to the lateral cross-sectional shape of a human's face. More preferably, the outlet opening comprises a strip- or sickle-shaped slit or a plurality of minor openings arranged in a strip- or sickle-type configuration over the upper end of the main body, to guide the airflow upward along the user's face in the fashion of a moving cloud of clean air or an air curtain along and around the user's face.

Regarding the construction of the main body, in an embodiment, a front part is configured to sit, when the apparatus is in use, on the chest of the user and which comprises at least one air duct and, in particular, comprises at least one fan and the control unit, and a rear part which is, when the apparatus is in use, placed on the user's back and which, in particular comprises the power supply unit. More specifically, the front part and rear part of the main body are connected by hinges or flexible elements which can contain at least one channel for a power supply line.

Regarding the material from which the apparatus body is formed, at present an embodiment wherein the main body is formed of plastic injection moulded parts appears as favourable. However, in other embodiments the main body or at least a portion thereof is formed of a flexible textile material which, as a matter of fact, has to be air-tight.

For further improving the performance of the proposed apparatus, in further embodiments at least a portion of the at least one air duct comprises a reflective and/or photocatalytic coating, and an additional UV emitter emitting radiation of an appropriate wavelength is provided and arranged such that it emits radiation to the portion which has the reflective and/or photocatalytic coating, to improve the antiviral effect and/or to add a self-cleaning effect.

A further improvement can be reached in a configuration, which within the air duct or at the inlet end thereof a particle filter is provided.

In further embodiments with enhanced disinfection performance, within the air duct or at the inlet end thereof a dielectric barrier unit or corona discharge unit is provided, to generate ozone from the ambient air.

Further aspects and advantages of the invention become apparent in the attached figures, in which
- Fig. 1: is a schematic side view of an embodiment of the apparatus, worn by a user,
- Fig. 2: a perspective view of an embodiment of the apparatus,
- Fig. 2A: a detail view of the apparatus shown in Fig. 2,
- Fig. 3: a schematic illustration of a further embodiment of the apparatus, as a partial cross-sectional front view, and
- Fig. 4: a block diagram of essential components of a further embodiment of the invention.

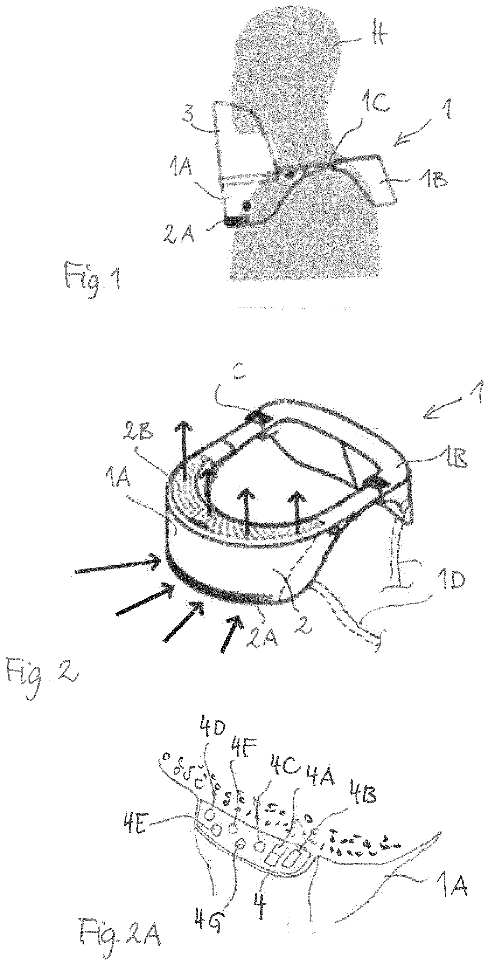

Fig. 1 is a side view of a wearable disinfection apparatus 1, worn by a human user H on his shoulders, and Fig. 2 is a perspective view of the apparatus 1, seen from above. Fig. 2A is a detail view of a modified apparatus 1', which has a multifunctional operating and display board.

The apparatus of Figs. 1 to 2A comprises a front part 1A which has, seen from above, basically a U-shape and is dimensioned such that it surrounds the neck of the user H and rests on both his shoulders and his chest. A rear part 1B is connected to the front part 1A by elastic members 1C on each free end of the "U" of the front part A. The rear part 1B is configured to rest on the rear part of the user's shoulders and upper part of his back. Both parts 1A and 1B can be made as injection-moulded plastic parts.

As can be seen in Fig. 2, the front part 1A has, at its lower end, a slit-shaped inlet opening 2A of an internal air duct 2 (indicated with a dashed line) and, in its upper surface, as an outlet opening 2B of the air duct, a sickle-shaped arrangement of many minor openings. Through the inlet port 2A, by means of a fan (not shown) ambient air A is sucked into the air duct 2, and through the outlet opening 2B a curtain-shaped flow of disinfected air DA is discharged.

The airflow of disinfected air DA flows from below the chin upwards to the mouth and nose of the user and along basically his whole face. It terms a "moving cloud" of clean air which in the following is designated as "air curtain". Thus, it acts as an invisible "mask" protecting him from inhaling un-disinfected air from the surround atmosphere.

In Fig. 1, it is shown that a clear face shield 3 is mounted to the upper surface of the front part 1A. This face shield 3 can further protect the user from being hit by polluted air (e.g. if it is heavily windy) and on the other hand serves for protecting other humans against the user of the apparatus, in case he sneezes or very strongly exhales.

In Fig. 2 an optional arrangement of straps 1D is shown, which can be mounted to the front 1A and rear part 1B and can be connected with each other, for better fixation of the apparatus 1 on the shoulder of the user, giving him more freedom to move, when wearing the apparatus.

Fig. 2A shows, in a detail view, an operating and display board 4 of a modified disinfection apparatus 1', which is arranged on an extension of the upper surface of the front part 1A'. As an exemplary configuration, it is shown that the operating and display board 4 comprises an on/off switch 4A, an USB socket 4B for recharging a battery (not shown) of the apparatus, and a charge status LED 4C (e.g. a red LED).

Furthermore, two LEDs 4D, 4E (e.g. green LEDs) for indicating two possible operation states of UV-C emitters (not shown) in the apparatus, and two further LEDs 4F, 4G (e.g. blue LEDs) for indicating two operation states of the fan (likewise not shown) in the apparatus are provided. In this embodiment, the battery is arranged preferably within the extension of the front part of the apparatus. In other embodiments, the USB socket and the battery can be arranged in other positions in the apparatus 1, e.g. in the rear part 1B.

The components mentioned herein above and the function thereof, in an exemplary embodiment, are explained below.

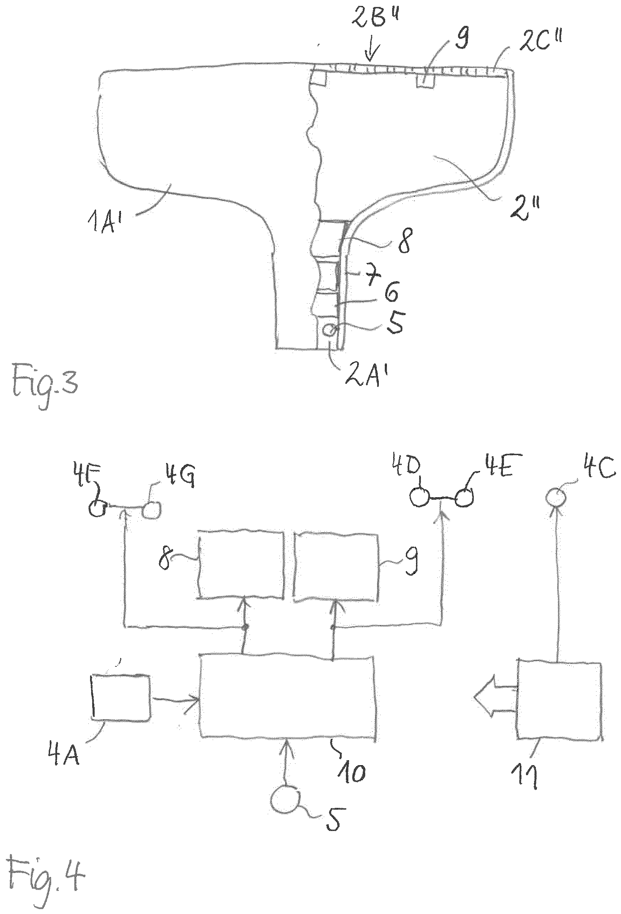

Fig. 3 is a front view, partially cut away, of a further modified apparatus 1". The apparatus 1" differs from the apparatus 1 in Figs. 1 and 2 insofar, as the lower portion of the front part 1A" is shaped differently and has a circular inlet opening 2A" in the lower end surface of the front part 1A". As can be seen in the cut-away part of the figure, in this modified inlet opening 2A" a humidity sensor 5 is arranged for determining the humidity of the ambient air.

Furthermore, above the inlet opening a particle filter unit 6 is provided, for cleaning the incoming ambient air from dust and larger liquid droplets. Above the filter unit 6, a corona discharge unit 7 is arranged for further enhancing the performance of the disinfection apparatus by generating ozone and mixing it into the air curtain which is discharged through the outlet opening 2B". The airflow between the inlet opening 2A" and the outlet opening 2B" of the air duct 2" is generated by means of a fan 8 which is arranged in the inlet portion of the front part of the apparatus, above the corona discharge unit 7.

On the lower surface of a perforated plate 2C" which forms the upper end of the front part of the apparatus, three UV-C LEDs 9 are arranged such that they emit UV-C radiation into the air duct 2". The inner surface of the air duct 2" has a reflective photocatalytically active coating 2D" which serves for improving the usage of the UV-C radiation by providing multiple reflections thereof. In addition to this, a photocatalytically active coating 2D can be provided in combination with an UV-A emitter, for adding a photocatalytic cleaning effect to the basic disinfection effect.

Fig. 4 is a block diagram which illustrates an exemplary structure and function of those components which are important for the active operation of the apparatus 1, 1' or 1", respectively. It is important to note that the following explanation of sensors, control components and control modes is, as likewise the explanations further above, just exemplary. Many other modes of operating an apparatus according to the invention and of using several sensors, manual actuators and display indicators are within the scope of the present invention.

The on/off switch 4A is, in this embodiment, the only manual actuating element of the apparatus and activates a control unit 10 which controls the operation of the fan 8 and the UV-C emitters 9 in dependence on signals which the control unit receives from the humidity sensor 5. For example, the operation of the fan 8 can be controlled according to two selectable speeds, and the operation of the UV-C emitters can be controlled in two different intensities, to optimize the disinfecting treatment of incoming air in line with the humidity thereof. Corresponding to the selected operation mode of the fan 8 and the UV-C LEDs 9, the controller sends signals to the LEDs 4D-4G, for indicating the operation status to the user.

All electronic components are provided with electrical energy from a rechargeable battery 11 through power supply lines which are omitted in the figure. A low charge status of the battery 11 is indicated by the charge status indicator LED 4C.

## Claims

1. Wearable apparatus for breathing air disinfection, the apparatus comprising:
- a wearable main body forming at least one ambient air duct which has an inlet end and an outlet end, which is configured to be worn by a human user on her/his shoulder or neck, and, when in use, the outlet of the air duct is below the chin of the user, wherein the air duct is configured such that it guides the air flow generated by the at least one fan at least to mouth and nose of the user,
- at least one UV-C radiation emitter, arranged within the air duct or at an end thereof such that it emits UV-C radiation into the air duct,
- at least one fan which is arranged within the air duct or at one end thereof, which generates an airflow from the inlet end to the outlet end,
- a power supply unit for supplying electrical power to the at least one UV-C emitter and at least one fan, arranged within or on the main body, and
- a control unit for controlling the operation of the at least one UV-C emitter and at least one fan, arranged within or on the main body.

2. Apparatus of claim 1, wherein the main body is configured, when in use, to extend from below the chin to the chest and/or at least one shoulder or the back of the user, and the inlet opening is located close to the chest or shoulder or back of the user.

3. Apparatus of claim 2, wherein the main body is configured to rest, in an upright position of the user, on her/his shoulder or neck, wherein the main body in particular comprises fixation means, such as belts or straps, for fixing it to the shoulder or neck of the user even in a non-upright position.

4. Apparatus of one of the preceding claims, wherein at least that portion of the main body which is close to the chin of the user has a curved lateral cross-sectional shape to adapt to the lateral cross-sectional shape of a human's face.

5. Apparatus of one of the preceding claims, wherein the outlet opening comprises a sickle-shaped slit or plurality of minor openings arranged in a strip-shaped, in particular sickle-type, configuration over the upper end of the main body, to guide the airflow upward along and around the user's face in an air curtain.

6. Apparatus of one of the preceding claims, wherein the main body comprises:
a front part configured to sit, when the apparatus is in use, on the chest of the user and which comprises at least one air duct and, in particular,
comprises at least one fan and the control unit, and
a rear part which is, when the apparatus is in use, placed on the user's back and which, in particular, comprises the power supply unit.

7. Apparatus of claim 6, wherein the front part and rear part of the main body are connected by hinges or flexible elements which contain at least one channel for a power supply line.

8. Apparatus of one of the preceding claims, wherein the main body is formed of plastic injection moulded parts or at least a portion thereof is formed of a flexible textile material.

9. Apparatus of one of the preceding claims, wherein at least a portion of the at least one air duct comprises a reflective and/or photocatalytic coating, and the UV-C emitter or, in of a photocatalytic coating, an UV-A emitter is provided in the air duct and arranged such that it emits radiation to the portion which has the reflective and/or photocatalytic coating, to provide an additional antiviral and/or self-cleaning effect.

10. Apparatus of one of the preceding claims, wherein within the air duct or at the inlet end thereof a particle filter is provided.

11. Apparatus of one of the preceding claims, wherein within the air duct or at the inlet end thereof a dielectric barrier unit or corona discharge unit is provided, to generate ozone from the ambient air, to enhance the disinfection effect provided by the apparatus.

12. Apparatus of one of the preceding claims, comprising a clear plastic face shield, removably arranged on the upper end of the main body, relative to the face of the user outside of the outlet opening of the air duct.

13. Apparatus of one of the preceding claims, wherein an operating and display board is provided on the main body, the board comprising actuator means for manually controlling the operation and display means for indicating the operation status of the apparatus and the charge status of the power supply unit.

14. Apparatus of one of the preceding claims, comprising at least one of the following sensors: UV-C light sensor, airflow sensor, humidity sensor, power supply voltage sensor, and temperature sensor, and the control unit is adapted to receive and process signals from the at least one sensor, to control the operation of the apparatus, in particular of the at least one UV-C emitter and the at least one fan.
